# EUROPEAN PATENT APPLICATION

(11) **EP 4 169 902 A1**
(43) Date of publication of application: **26.04.2023**
(21) Application number: 21382962.5
(22) Date of filing: 25.10.2021
(51) Int. Cl.: C07C 215/50, C07C 215/64, A61P 39/02, C07C 217/70

(54) **SPERMINE DERIVATIVES AS HEPARIN ANTIDOTES**

(71) Applicant: Consejo Superior de Investigaciones Científicas (CSIC), 28006 Madrid (ES); Institut Químic de Sarrià CETS Fundació Privada, 08017 Barcelona (ES)
(72) Inventor: Alfonso Rodriguez, Ignacio, 08034 Barcelona (ES); Carbajo López, Daniel, 08034 Barcelona (ES); Pérez Ruiz, Yolanda, 08034 Barcelona (ES); Bujons Vilás, Jordi, 08034 Barcelona (ES); Prats Miravitllas, Eva, 08034 Barcelona (ES); Guerra Rebollo, Marta, 08017 Barcelona (ES)
(74) Representative: Pons

(57) **Abstract**

The invention relates to new spermine derivatives of Formula **I**, wherein the meaning of the groups R₁, R₂, n and m is defined in the description, to a pharmaceutical composition thereof and to their use as heparin antidotes in the control of blood coagulation.

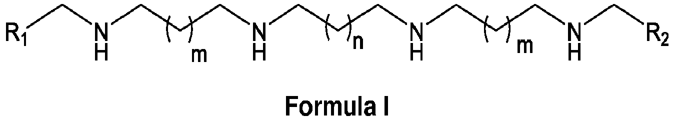

## Description

The invention relates to new spermine derivatives of Formula I, to their pharmaceutical composition thereof and to their use as antidote of heparin in the control of blood coagulation.

### BACKGROUND ART

Heparin (Hep) is a highly anionic glycosaminoglycan (GAG) extensively used in clinics as anticoagulant (Gandhi, N. S. & Mancera, R. L. Heparin/heparan sulphate-based drugs. Drug Discov. Today 15, 1058-1069 (2010) and Weitz, J. & Harenberg, J. New developments in anticoagulants: Past, present and future. Thromb. Haemost. 117 7, 1283-1288 (2017)). For a safer usage of Hep, the accessibility to a family of efficient reversal agents is desirable, especially for cases of overdose, life-threatening bleeding or urgent high-risk surgery. However, the number of antidotes for heparin-type drugs is quite limited: in practice it is reduced to the administration of protamine (Guo, X., Han, I. S., Yang, V. C. & Meyerhoff, M. E. Homogeneous Enzyme-Based Binding Assay for Studying Glycosaminoglycan Interactions with Macromolecules and Peptides. Anal. Biochem. 235, 153-160 (1996)), a small arginine-rich nuclear protein. Recent reports on other alternative macromolecules to reverse heparin drugs include a polymeric polycationic dendrimer (UHRA) (Kalathottukaren, M. T. et al. A Polymer Therapeutic Having Universal Heparin Reversal Activity: Molecular Design and Functional Mechanism. Biomacromolecules 18, 3343-3358 (2017)), a monoclonal antibody (idarucizumab) (Pollack, C. V et al. Idarucizumab for Dabigatran Reversal - Full Cohort Analysis. N. Engl. J. Med. 377, 431-441 (2017)), a modified version of the recombinant human Xa coagulation factor (andexanet alpha) (Connolly, S. J. et al. Full Study Report of Andexanet Alfa for Bleeding Associated with Factor Xa Inhibitors. N. Engl. J. Med. 380, 1326-1335 (2019)), as well as peptides (Li, T. et al. New synthetic peptide with efficacy for heparin reversal and low toxicity and immunogenicity in comparison to protamine sulfate. Biochem. Biophys. Res. Commun. 467, 497-502 (2015)) and peptidomimetics (Montalvo, G. L. et al. De Novo Design of Self-Assembling Foldamers That Inhibit Heparin-Protein Interactions. ACS Chem. Biol. 9, 967-975 (2014)) mimicking protamine.

Considering the general problems of using high molecular weight drugs (Schuster, J. et al. In Vivo Stability of Therapeutic Proteins. Pharm. Res. 37, 23 (2020), the search of alternative small molecules able to revert the anticoagulation effects of heparin is a hot topic in current research. Among these small-molecules, surfen (Schuksz, M. et al. Surfen, a small molecule antagonist of heparan sulfate. Proc. Natl. Acad. Sci. 105, 13075 LP - 13080 (2008)) and ciraparantag (also named PER977) (Ansell, J. E. et al. Use of PER977 to Reverse the Anticoagulant Effect of Edoxaban. N. Engl. J. Med. 371, 2141-2142 (2014) and Ansell, J. E. et al. Ciraparantag safely and completely reverses the anticoagulant effects of low molecular weight heparin. Thromb. Res. 146, 113-118 (2016)) would be the most representative examples.

The bis(quinolyl)urea surfen is an approved drug originally used as an excipient for the production of depot insulin that has been recently identified as a heparan sulfate binder (Weiss, R. J. et al. Small molecule antagonists of cell-surface heparan sulfate and heparin-protein interactions. Chem. Sci. 6, 5984-5993 (2015)). However, the oncogenicity of surfen at the doses needed to observe effects on the coagulation rate has precluded further development in this specific field (Huner, D. T. & Hill, J. M. Surfen: A Quinoline with Oncogenic and Heparin-Neutralizing Properties. Nature 191, 1378-1379 (1961)).

On the contrary, the cationic synthetic ciraparantag molecule is currently in clinical trials as inhibitor of different anticoagulants (Kustos, S. A. & Fasinu, P. S. Direct-Acting Oral Anticoagulants and Their Reversal Agents-An Update. Medicines 6, (2019)). The actual mechanism of action of ciraparantag is still unclear probably due to the highly anionic charge density and polar nature of the heparin molecule, which makes extremely difficult its molecular recognition in highly solvating aqueous and ionic media. This chemical complexity is also accompanied by a large conformational flexibility in solution (Capila, I. & Linhardt, R. J. Heparin-Protein Interactions. Angew. Chemie Int. Ed. 41, 390-412 (2002)), making difficult to establish a preferred three-dimensional structure either by experimental (X-ray diffraction, NMR) or theoretical (molecular modeling) approaches (Khan, S., Gor, J., Mulloy, B. & Perkins, S. J. Semi-Rigid Solution Structures of Heparin by Constrained X-ray Scattering Modelling: New Insight into Heparin-Protein Complexes. J. Mol. Biol. 395, 504-521 (2010)) Recently, a heparin inhibitor has been identified using a dynamic combinatorial chemistry screening protocol (Corredor, M., Carbajo, D., Domingo, C., Perez, Y., Bujons, J., Messeguer, M. & Alfonso, I. Dynamic Covalent Identification of an Efficient Heparin Ligand. Angew. Chemie - Int. Ed. 57, 11973-11977 (2018), which is structurally related to spermine, opening the possibility to use spermine derivatives for this specific application. However, for a suitable in vivo application, more potent antidotes with submicromolar activity are desirable to minimize the need of high doses for clinical usage.

Considering all the above, it would be desirable to have improved heparin antidotes with good anticoagulant rates, based on simple, easy to produce synthetic small molecules, more potents for a suitable in vivo application with submicromolar activity in order to minimize the need of high doses.

### SUMMARY OF THE INVENTION

A first aspect of the present invention relates to a compound of Formula I: 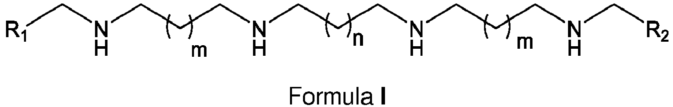 wherein:
n is an integer between 0 and 3;
each m is independently an integer between 0 and 3;
R₁ is phenyl, naphthyl or fluorenyl, wherein R₁ is optionally substituted by one to three groups R₃;
R₂ is naphthyl or fluorenyl, wherein R₂ is optionally substituted by one to three groups R₃;
each R₃ is independently -OH, -OR₄, halogen, -C₁₋₄ alkyl, -CHO or -OPO(OH)₂; and
R₄ is C₁₋₄ alkyl,
with the proviso that:
if R1 is 2-methoxyphenyl, 2-hydroxyphenyl or 2-methoxy-1-naphthalen-1-yl, n=2 and m=1, then R2 is not 1-naphthyl or 2-methoxy-1-naphthalen-1-yl; and
the compound of Formula I is not
   N1 ,N1'-(butane-1,4-diyl)bis(N3-(naphthalen-1-ylmethyl)propane-1,3-diamine, or
   N1,N1'-(butane-1,4-diyl)bis(N3-(naphthalen-2-ylmethyl)propane-1,3-diamine.

In one embodiment the invention relates to the compound of Formula I as defined above, wherein R₁ is phenyl, 1-naphthyl, 2-naphthyl or fluorenyl and R2 is 1-naphthl, 2-naphthyl or fluorenyl.

In another embodiment the invention relates to the compound of Formula I as defined above, wherein each R₁ and R₂ are independently substituted by one to three R₃ groups, and preferably by one R₃ groups.

In another embodiment the invention relates to the compound of Formula I as defined above, wherein each R₁ and R₂ are independently substituted by three R₃ groups.

In another embodiment the invention relates to the compound of Formula I as defined above, wherein R₁ is selected from

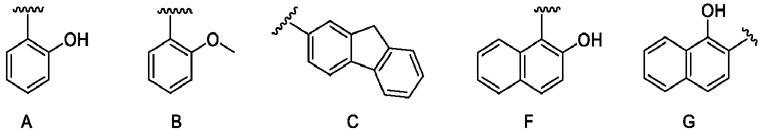

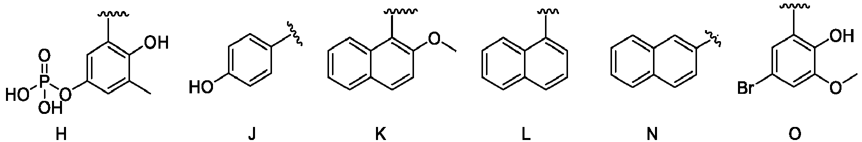

In another embodiment the invention relates to the compound of Formula I as defined above, wherein R₂ is selected from

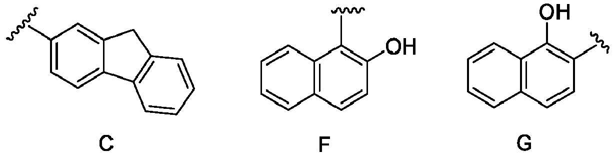

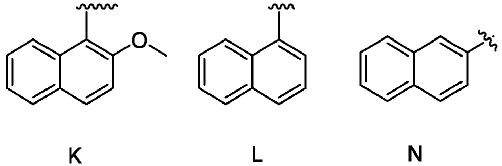

In another embodiment the invention relates to the compound of Formula I as defined above, wherein n is 2.

In another embodiment the invention relates to the compound of Formula I as defined above, wherein m is 1.

In another embodiment the invention relates to the compound of Formula I as defined above, wherein R₄ is -CH₃.

In another embodiment the invention relates to the compound of Formula I as defined above, wherein:
n is 2;
m is 1; and
R₄ is -CH₃.

In another embodiment the invention relates to a compound of Formula I selected from:

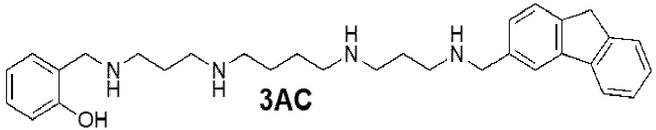

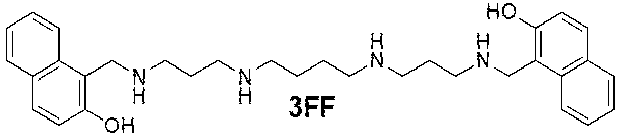

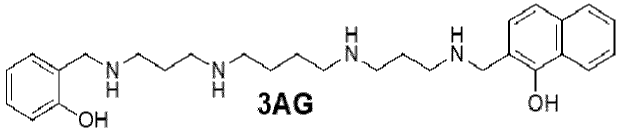

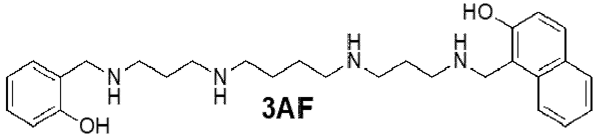

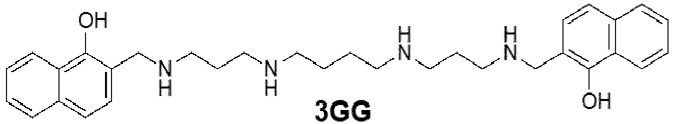

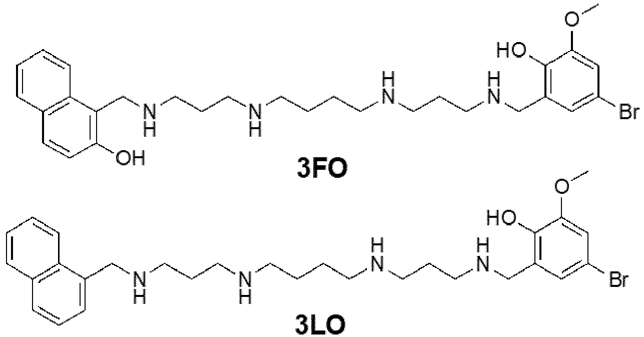

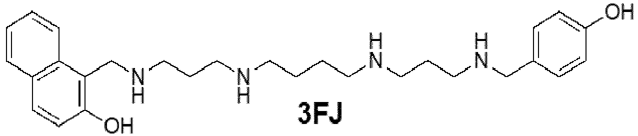

In another preferred embodiment the invention relates to a compound of Formula **I** selected from **3AC, 3FF, 3AG** and **3AF.** In a more preferred embodiment the compound of Formula **I** is selected from **3AC** and **3FF.**

Another aspect of this invention relates to a pharmaceutical composition, which comprises a compound of Formula **I** as described above, preferably which comprises a compound of Formula **I** selected from **3AC, 3FF, 3AF** and **3AG,** more preferably from **3AC** and **3FF,** or a pharmaceutically acceptable salt thereof and one or more pharmaceutically acceptable excipients.

Another aspect of the present invention relates to a compound of Formula **I**, as defined above, or a pharmaceutically acceptable salt thereof for use as a medicament.

In another embodiment the invention relates to a compound of Formula **I** selected from **3AC, 3FF, 3AF** and **3AG,** preferably from **3AC** and **3FF,** or a pharmaceutically acceptable salt thereof for use as a medicament.

Another aspect of the present invention relates to a compound of Formula **I**, as defined above, or a pharmaceutically acceptable salt thereof for use as antidote of heparin in the control of blood coagulation.

Another aspect of the present invention relates to a compound of Formula **I**, as defined above, preferably to a compound of Formula **I** selected from **3AC, 3FF, 3AF** and **3AG,** more preferably to a compound of Formula **I** selected from **3AC** and **3FF,** or a pharmaceutically acceptable salt thereof for use as antidote of heparin in the control of blood coagulation.

Another aspect of the present invention relates to a compound of Formula **I**, as defined above, preferably to a compound of Formula **I** selected from **3AC, 3FF, 3AF** and **3AG,** preferably to a compound of Formula **I** selected from **3AC** and **3FF,** or a pharmaceutically acceptable salt thereof for the manufacture of a medicament for use as antidote of heparin in the control of blood coagulation.

Another aspect of the present invention relates to the use of a compound of Formula **I**, as defined above, preferably to a compound of Formula **I** selected from **3AC, 3FF, 3AF** and **3AG,** more preferably to a compound of Formula **I** selected from **3AC** and **3FF,** or a pharmaceutically acceptable salt thereof for the manufacture of a medicament.

Another aspect of the present invention relates to a method of treating or preventing blood coagulation in a subject in need thereof, especially a human being, which comprises administering to said subject an effective amount of a compound of Formula **I,** as defined above, preferably to a compound of Formula **I** selected from **3AC, 3FF, 3AF** and **3AG,** more preferably to a compound of Formula **I** selected from **3AC** and **3FF,** or a pharmaceutically acceptable salt thereof.

The compounds of the present invention contain one or more basic nitrogen and may, therefore, form salts with organic or inorganic acids. Examples of these salts include: salts with inorganic acids such as hydrochloric acid, hydrobromic acid, hydroiodic acid, nitric acid, perchloric acid, sulfuric acid or phosphoric acid; and salts with organic acids such as methanesulfonic acid, trifluoromethanesulfonic acid, ethanesulfonic acid, benzenesulfonic acid, p-toluenesulfonic acid, fumaric acid, oxalic acid, acetic acid, maleic acid, ascorbic acid, citric acid, lactic acid, tartaric acid, malonic acid, glycolic acid, succinic acid and propionic acid, among others. Some of the compounds of the present invention may contain one or more acidic protons and, therefore, they may also form salts with bases. Examples of these salts include salts with inorganic cations such as sodium, potassium, calcium, magnesium, lithium, aluminum, zinc, etc; and salts formed with pharmaceutically acceptable amines such as ammonia, alkylamines, hydroxylalkylamines, lysine, arginine, *N*-methylglucamine, procaine and the like.

There is no limitation on the type of salt that can be used, provided that these are pharmaceutically acceptable when used for therapeutic purposes. The term pharmaceutically acceptable salt refers to those salts which are, according to medical judgment, suitable for use in contact with the tissues of humans and other mammals without undue toxicity, irritation, allergic response and the like. Pharmaceutically acceptable salts are well known in the art.

The salts of a compound of Formula **I**, as defined above, particularly the salts of a compound of Formula **I** selected from **3AC, 3FF, 3AF** and **3AG** can be obtained during the final isolation and purification of the compounds of the invention or can be prepared by treating a compound **3AC, 3FF, 3AF** and **3AG** with a sufficient amount of the desired acid or base to give the salt in a conventional manner. The salts of the compounds **3AC, 3FF, 3AF** and **3AG** can be converted into other salts of the compounds of Formula **I** by ion exchange using ionic exchange resins.

The compounds **3AC, 3FF, 3AF** and **3AG** and their salts may differ in some physical properties but they are equivalent for the purposes of the present invention. All salts of the compounds **3AC, 3FF, 3AF** and **3AG** are included within the scope of the invention.

The compounds of the present invention may form complexes with solvents in which they are reacted or from which they are precipitated or crystallized. These complexes are known as solvates. As used herein, the term solvate refers to a complex of variable stoichiometry formed by a solute (a compound of Formula **I**, preferably selected from **3AC, 3FF, 3AF** and **3AG** or a salt thereof) and a solvent. Examples of solvents include pharmaceutically acceptable solvents such as water, ethanol and the like. A complex with water is known as a hydrate. Solvates of compounds of the invention (or salts thereof), including hydrates, are included within the scope of the invention.

The compounds of Formula **I**, preferably **3AC, 3FF, 3AF** and **3AG** may exist in different physical forms, i.e. amorphous and crystalline forms. Moreover, the compounds of the invention may have the ability to crystallize in more than one form, a characteristic which is known as polymorphism. Polymorphs can be distinguished by various physical properties well known in the art such as X-ray diffraction pattern, melting point or solubility. All physical forms of the compounds **3AC, 3FF, 3AF** and **3AG,** including all polymorphic forms ("polymorphs") thereof, are included within the scope of the invention.

The present invention also relates to a pharmaceutical composition that comprises a compound of the present invention (or a pharmaceutically acceptable salt or solvate thereof) and one or more pharmaceutically acceptable excipients. The excipients must be "acceptable" in the sense of being compatible with the other ingredients of the composition and not deleterious to the recipients thereof.

The compounds of the present invention can be administered in the form of any pharmaceutical formulation, the nature of which, as it is well known, will depend upon the nature of the active compound and its route of administration. Any route of administration may be used, for example oral, parenteral, nasal, ocular, rectal and topical administration.

Injectable preparations, according to the present invention, for parenteral administration, comprise sterile solutions, suspensions or emulsions, in an aqueous or non-aqueous solvent such as propylene glycol, polyethylene glycol or vegetable oils. These compositions can also contain coadjuvants, such as wetting, emulsifying, dispersing agents and preservatives. They may be sterilized by any known method or prepared as sterile solid compositions, which will be dissolved in water or any other sterile injectable medium immediately before use. It is also possible to start from sterile materials and keep them under these conditions throughout all the manufacturing process.

In the above definitions, the term C₁₋₄alkyl, as a group or part of a group, means a straight or branched alkyl chain which contains from 1 to 4 carbon atoms and includes the groups methyl, ethyl, propyl, isopropyl, butyl, isobutyl, *sec*-butyl and *tert*-butyl.

Halogen means fluoro, chloro, bromo or iodo.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings are provided by way of illustration and are not intended to be limiting of the present invention.

### BRIEF DESCRIPTION OF THE DRAWINGS

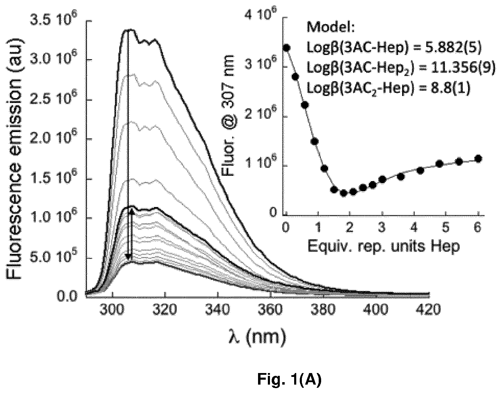

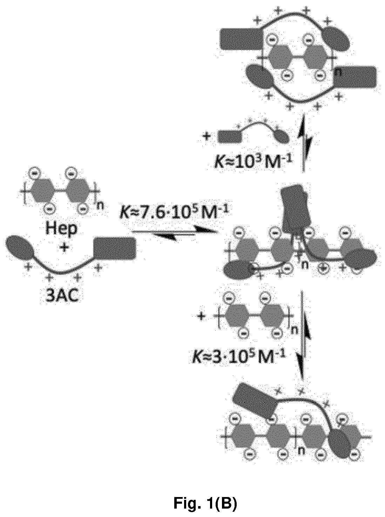

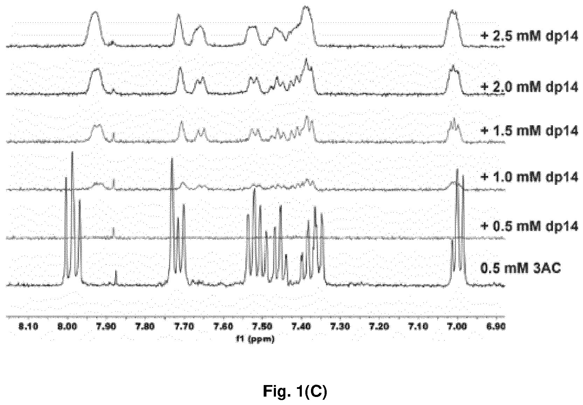

**Fig. 1****. (A)** luorescence emission of 3AC upon titration with heparin. The inset shows the variation of the emission fluorescence at 307 nm against the equivalents of repeating units of Hep (with the fitting to the proposed binding model). **(B)** Schematic representation of the binding model between 3AC and Hep, with the corresponding stepwise equilibrium constants shown for each step. **(C)** Stacked partial ¹H NMR spectra of 3AC upon increasing addition of dp14 heparin (concentration of dp14 related to the disaccharide repeating unit).

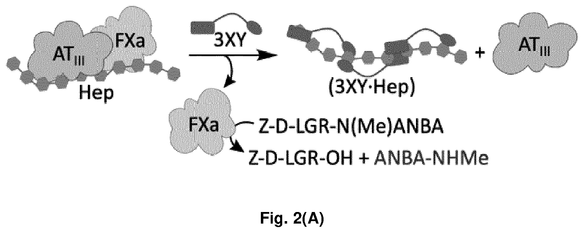

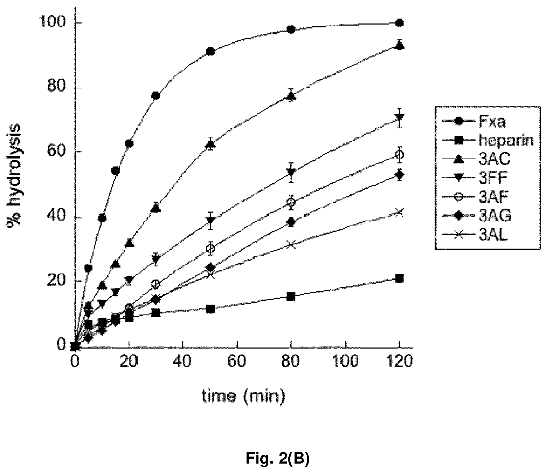

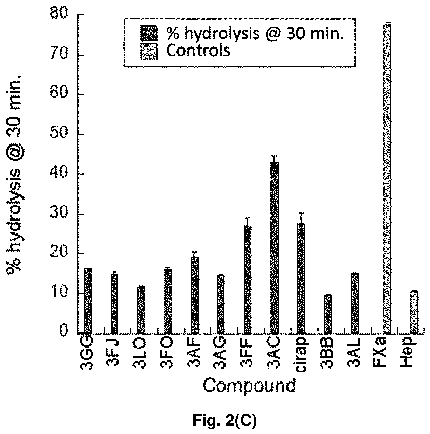

**Fig. 2****. (A)** Schematic representation of the in vitro enzymatic assay used to test the reversal effect of the heparin ligands. **(B)** Plot of the time evolution of the hydrolysis of the peptide substrate (485 µM) under different conditions: all traces (0.085 µg/mL of FXa and 0.02 IU/mL of AT_{III}), all traces except solid circles (0.1 IU/mL of Hep), traces with symbols except "X" and solid circles (1 µM of each ligand, legend in the inset). **(C)** Plot of the percent hydrolysis at 30 min. for some selected ligands, including the FXa and Hep control tests, as well as the results obtained with ciraparantag (cirap.).

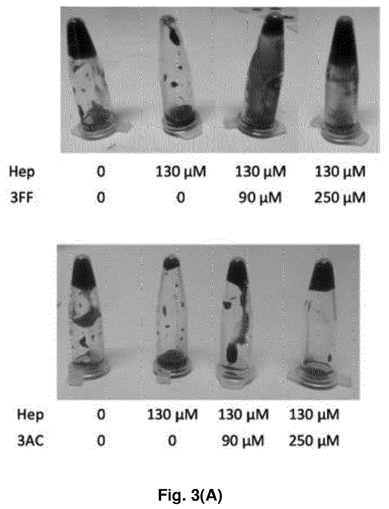

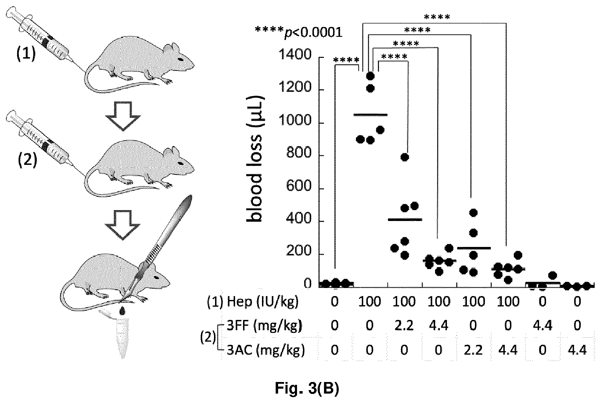

**Fig. 3****. (A)** Ex vivo coagulation test with freshly extracted mouse blood using Hep in combination with 3FF and 3AC antidotes. Molar concentrations of Hep are related to the disaccharide repeating units. **(B)** In vivo tail transection assay performed in mice to test the reversal of Hep activity by 3AC or 3FF. Statistically different (ANOVA) results (p<0.0001) are labelled.

### Examples

### General methods

**General:** Reagents and solvents were purchased from commercial suppliers (Aldrich, Fluka or Merck) and were used without further purification. Flash chromatographic purifications and preparative reversed-phase purifications were performed on a Biotage^{®}Isolera PrimeTM equipment. TLCs were performed using 6x3 cm SiO₂precoated aluminium plates (ALUGRAM^{®}SIL G/UV254)

**Nuclear Magnetic Resonance (NMR):** Spectroscopic experiments for the characterization of compounds were carried out on a Varian Mercury 400 instrument (400 MHz for ¹H and 101 MHz for ¹³C). Chemical shifts (δH) are quoted in parts per million (ppm) and referenced to the appropriate NMR solvent peak(s). 2D-NMR experiments COSY, HSQC and HMBC were used where necessary in assigning NMR spectra. Spin-spin coupling constants (J) are reported in Hertz (Hz). For the experiments performed in aqueous buffer a low molecular weight heparin was used: dp14 (from Iduron, prepared by high resolution gel filtration of partial heparin lyase digestion of high quality heparin, MW average ~ 4100). One- and two-dimensional (1D and 2D) NMR experiments were performed at 298 K on a 500 MHz Bruker AVANCEIII-HD equipped with a z-gradient (65.7 G cm⁻¹) inverse TCI-cryoprobe. Samples were dissolved in 5 mM Tris-d11 buffer with 50 mM NaCl (in D₂O, pH 7.5, uncorrected pH meter reading). Bruker TopSspin 3.5pl6 standard pulse sequences were used for 1D and 2D experiments.

**Liquid Chromatography coupled to Mass Spectrometry:** Analyses were carried out at the IQAC Mass Spectrometry Facility, using a UPLC-ESI-TOF equipment: [Acquity UPLC^{®}BEH C₁₈ 1.7 mm, 2.1×100 mm, LCT Premier Xₑ, Waters]. (CH₃CN + 20 mM HCOOH and H₂O + 20 mM HCOOH) mixtures at 0.3 mL/min were used as mobile phase.

**Surface Plasmon Resonance (SPR):** Affinity experiments between inhibitors and heparin were performed on an Open SPR^{™}(Nicoya). All measurements were performed at 25°C using a working buffer of 25 mM Tris at pH 7.5. Biotin-loaded sensor chips (NICOYA) were further functionalized with streptavidine (50 µg/mL) and later with biotin-heparin (50 µg/mL). Binding experiments to heparin were performed by injecting inhibitors at desired concentrations and at a rate of 40 µL/min. Between binding assays, the surface was regenerated by exposure to an injection of 10 mM HCl. Fitting has been performed by Trace Drawer software using a 'one-to-one two-state algorithm', which considers a 1:1 binding with a further equilibrium like a conformational change. Results obtained from three independent experiments at three different concentrations of the ligands were fit globally to render the corresponding on/off rate constants and the apparent dissociation constants (*K*_{D}^{app}, Table 1, as shown below).

**Fluorescence spectroscopy titration:** Fluorescence emission and excitation spectra were collected on a Photon Technology International Instrument, the Fluorescence Master Systems, using the Software Felix32 and cuvettes with 10 mm path length. Stock solutions of the corresponding binder (20 µM) and heparin (18 IU/mL) were prepared in 1 mM Bis-Tris buffer at pH 7.5. Then, 2 mL of the binder solution was placed on a quartz cell and the emission fluorescence spectrum was measured upon excitation at 280 nm. Then, small volumes of the heparin stock solution were added to the cell, and the fluorescence spectra was acquired after each addition. For 3AC, the titration experiments were fitted using HypeSpec2008 software, which allows a non-linear global fitting of the full emission spectra to a binding mode as defined by the user.

**Blood coagulation factor in vitro enzymatic assays:** Recombinant antithrombin III and coagulation Factor Xₐ were obtained from the Berichrom Heparin test, supplied by SIEMENS. Following the indications of the test kit, stock solutions were prepared as follows: Human antithrombin III (1 IU/mL), Factor Xₐ reagent (0.4 µg/mL, human plasma fraction with the additives Tris, sodium chloride and EDTA) and a chromogenic substrate specific for factor Xₐ (4 mM of Z-D-Leu-Gly-ArgANBA-methyl amide). On the other hand, 4-nitroaniline (Sigma Aldrich) was added to the substrate solution at a concentration of 1.6 mM and was used as internal standard to quantify the hydrolysis of the chromogenic substrate. Heparin (sodium salt from porcine intestinal mucosa, polydisperse, from 6000 to 30000 Daltons) was purchased from Sigma-Aldrich. All compounds were dissolved in miliQ water at the desired stock concentrations prior to start the assays and kept at 4ºC. Various concentrations of the ligand, heparin (0.1 IU/mL), human antithrombin III solution (3.5 µL) and Factor Xa reagent solution (35 µL) were added in order to an Eppendorf and brought to a total volume of 145 µL. Then, 20 µL of Reagent Substrate solution was added to start the experiment and the mixture was vigorously shaken at 25ºC. 20 µL samples were taken at minutes 5/10/15/20/30/50/80/120, diluted with 40 µL of acetic acid (20% v/v), and injected in the analytical HPLC. The gradient ranged from 5% of ACN (0.1% formic acid) in water (0.1% formic acid) to 100% of ACN in 24 minutes using a 15 × 4.6 mm KROMAPHASE C₁₈ 5.0 µm column (retention time of cleaved chromophore 9.1 min, retention time of chromogenic substrate 13.9min, retention time of 4-nitroaniline 15.4min). Concentrations of reagents have been adjusted compared with previous studies with the intention to slow down the total exhaustion of peptide substrate. In this way the activity can be more carefully modulated and any change is easier to detect. FXₐ/AT_{III} activity was represented as percent of hydrolysis, which was calculated from the normalized area of cleaved peptide at 405 nm at each corresponding time point. Experiment carried out in absence of heparin was considered as maximum of activity while experiment with heparin and no ligand was considered as negative control (maximum inhibition of FXₐ by heparin). Concentration of heparin was selected for the measurement to render a significant inhibition within experimental time, while allowing the reaction to proceed.

**Ex vivo blood coagulation assays:** Freshly collected mouse blood was collected and directly used without further treatment. 300 µL aliquots were prepared. To them, Hep (130 µM), and various concentration of ligand (3AC or 3FF) were added. The samples of the different conditions were added to an Eppendorf tube and a picture was taken after 15 minutes. The clot formation was confirmed by turning around the Eppendorf vials.

**In vivo tail transection assays with mice:** Mice were randomly assigned into 6 groups (n=6 for each group): Saline group (only saline was given), Hep group (Hep injection, 100 IU/kg), 3AC (two groups, Hep injection followed by injection of 3AC at 2.2 mg/kg or 4.4 mg/kg) and 3FF (two groups, Hep injection followed by injection of 3FF at 2.2 mg/kg or 4.4 mg/kg). Two additional control groups (n=3) were included with injection of just the ligand (3AC or 3FF) at the highest dose (4.4 mg/kg). Animals were anesthetized by i.p. injections of 0.465 mg/kg xylazine (Rompum; Bayer DVM) and 1.395 mg/kg ketamine (Imalgene; Merial Laboratorios). After anesthetization, mice were injected intravenously with different solutions depending on the group they were assigned. After 5 minutes, the scalpel was cleaned and disinfected, then each tail was transected at a site 5 mm from the end of tail with scalpel and immediately immersed in a tube containing 1 mL normal saline buffer at 37 °C. The blood draining out from the wound was collected in each tube. Tails were let bleed during 10 minutes. The total blood volume in each tube was quantified by spectrophotometry (absorbance at 414 nm) from a standard curve that was constructed with known volumes of blood hemoglobin concentration and corresponding absorbance value. Statistical analysis of the data was done with Kaleida Graph 4.5.4 using ANOVA.

### Synthetic protocols and characterization data

### General synthetic scheme for symmetric and asymmetric molecules:

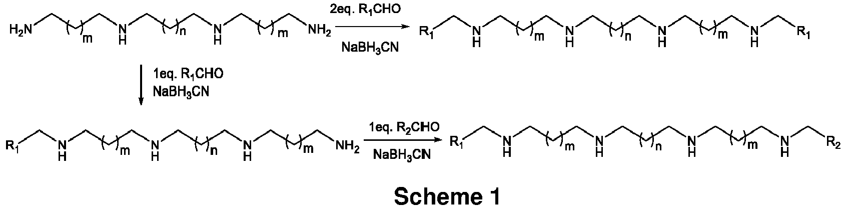

Compounds of Formula I may be prepared following different methods known by a person skilled in the field of organic synthesis. In particular, they may be obtained following the routes depicted in Scheme 1.

### Homomers:

First, spermine or any other suitable polyamine reacts with 2 eq. of an arylaldehyde (R1) dissolved, for example in THF, to give the corresponding imine derivative. The imine reacts with a reductive reactant, such as NaBH₃CN, to obtain the desired 3R₁R₁ compound of Formula **I**.

### Heteromers

First, spermine or any other suitable polyamine reacts with 1 eq. of an arylaldehyde (R1) to give the corresponding imine derivative. The imine reacts with a reductive reactant, such as NaBH₃CN, to obtain the desired 3R₁.

Then, intermediate 3R₁ compound dissolved in MeOH, is reacted with a second aryl aldehyde of interest **R₂**, affording another imine, which after reduction gives the desired 3R1R2 compound of Formula **I**.

Purification of intermediates and/or final compounds of Formula (I), if required, can be carried out by HPLC at semipreparative level.

### Detailed synthesis and characterization:

Compounds 3AA (2,2'-(2,6,11,15-tetraazahexadecane-1,16-diyl)diphenol), 3BB (*N*¹,*N*^{1'}-(butane-1,4-diyl)bis(*N*³-(2-methoxybenzyl)propane-1,3-diamine)) and 3AL (2-(16-(naphthalen-1-yl)-2,6,11,15-tetraazahexadecyl)phenol), used for comparative purposes were previously characterized in Carbajo, D., Perez, Y., Bujons, J. & Alfonso, I. Live-Cell-Templated Dynamic Combinatorial Chemistry. Angew. Chemie - Int. Ed. 59,17202-17206 (2020).

### Intermediates 3R1:

### 2-(((3-((4-((3-aminopropyl)amino)butyl)amino)propyl)amino)methyl)phenol (3A)

Spermine (120mg, 0.594mmol) was solved in 75mL of THFₐₙₕ at 0ºC. 2-hydroxybenzaldehyde (58µL, 0.534mmol) solved in 20mL of MeOH was dropwise added and the reaction was stirred overnight. The day after, NaBH₃CN (67mg, 1.07mmol) was added and stirred 24h. Reaction was stopped by addition of 3mL of H₂O and 3mL of HCI 1M. THF was carefully rotavapored and reaction crude was purified by reverse phase chromatography with a gradient of ACN (0.1% TFA) and water (0.1% TFA). 77mg of **3A** were obtained as white powder (Yield => 47%, Purity => 91.1%).
**¹H RMN (H₂O/D₂O):** δ 7.2 (2H, m), 6.85 (2H, m), 4.11 (2H, s), 2.97 (12H, m), 1.94 (4H, m), 1.61 (4H, m).¹³**C RMN (H₂O/D₂O)**: δ 154.9, 131.7, 131.5, 120.6, 115.5, 114.8, 46.9 ,44.4 (, 43.6, 36.4, 23.6, 22.6, 22.4.
**MS:** Calculated for C₁₇H₃₂N₄O: 308.4700; found: 309.2684 (M + H)⁺

### 4-(((3-((4-((3-aminopropyl)amino)butyl)amino)propyl)amino)methyl)phenol (3J)

Following an analogous procedure to that described for 3A but using 4-hydroxybenzaldehyde, intermediate **3J** was obtained as white powder (Yield => 66%, Purity => 98.1%).
**¹H RMN (H₂O/D₂O)**: δ 7.18 (2H, d, J=8Hz), 6.78 (2H, d, J=8Hz), 3.95 (2H, s), 2.92 (12H, m), 1.89 (4H, m), 1.58 (4H, m). ¹³**C RMN (H₂O/D₂O):** 156.7, 131.4, 123.3, 115.9, 50.8, 47.1, 47.0, 44.8 (C10), 44.6, 43.9, 36.7, 24.2, 23.3, 23.2.
**MS:** Calculated for C₁₇H₃₂N₄O: 308.4700; Found MS => 309.2698 (M + H)⁺

### 2-(((3-((4-((3-aminopropyl)amino)butyl)amino)propyl)amino)methyl)-4-bromo-6-methoxyphenol (3O)

Spermine (54mg, 0.267mmol) was solved in 25mL of THFₐₙₕ at 0 ºC. 5-bromo-2-hydroxy-3-methoxybenzaldehyde (54mg, 0.24mmol) solved in 10mL of MeOH was dropwise added and the reaction was stirred overnight. The day after, NaBH₃CN (30mg, 0.48mmol) was added and stirred 24h. Reaction was stopped by addition of 2mL of H₂O and 2mL of HCl 1M. THF was carefully rotavapored and reaction crude was purified by reverse phase chromatography with a gradient of ACN (0.1% TFA) and water (0.1% TFA). 30mg of **3O** were obtained as brown powder (Yield => 31%, Purity => 97.3%).
**1H RMN (H2O/D2O):** δ 7.18 (1H, s), 7.01 (1H, s), 4.11 (2H, s), 3.77 (3H, s), 2.98 (12H, m), 1.96 m (4H, H9, m), 1.65 (4H, m)
13C RMN (H2O/D2O):δ 148.4, 144.0, 125.2, 118.5, 116.6, 111.1, 56.3,46.9,45.8, C14), 44.4, 44.3, 36.4, 23.6, 22.7, 22.4.
**MS:** Calculated C₁₈H₃₃BrN₄O₂: 416.1787; found: 419.1703 and 417.1704 (M + H)+

### Compounds of 3R1R1 type

### 1,1'-(2,6,11,15-tetraazahexadecane-1,16-diyl)bis(naphthalen-2-ol) (3FF)

Spermine (60 mg, 0.297 mmol) was solved in 25mL of MeOH. 2-hydroxy-1-naphthaldehyde (193 mg, 1.039 mmol) was then added solved in 12mL of MeOH. The solution was stirred 6h. Then, NaBH₃CN (126 mg, 2 mmol) was added and the reaction was stirred 24h. After addition of H₂O (2mL) and HCl 1M (2mL), the reaction was stirred 1h. Solvent was removed by rotavaporation. Reaction mixture was purified by reverse phase chromatography with a gradient of ACN (0.1% TFA) and water (0.1% TFA) to yield 78 mg (51%) of pure product **3FF** (99% by HPLC).
**¹H RMN (H₂O/D₂O)**: δ 7.86 (m, 6H), 7.54 (t, 2H, J₁= 8Hz), 7.36 (t, 2H, J₂=8Hz), 7.18 (d, 2H, J₃=12Hz), 4.65 (s, 2H), 3.14 (m, 4H), 3.00 (m, 8H), 2.04 (m, 4H), 1.62 (m, 4H).
¹³**C RMN (H₂O/D₂O)**: 154.1, 132.4, 132.1, 128.9, 128.4, 127.8, 123.7, 121.6, 117.3,46.8, 44.4, 43.8, 41.6, 22.6, 22.4.
**MS:** Calculated for C₃₂H₄₂N₄O₂: 514.7140; found: 515.3389 (M + H)⁺

### 2,2'-(2,6,11,15-tetraazahexadecane-1,16-diyl)bis(naphthalen-1-ol) (3GG)

Spermine (20mg, 0.099mmol) was solved in 5mL of MeOH at 0 ºC. 1-hydroxy-2-naphthaldehyde (40mg, 0.215mmol) was carefully added solved in 2mL of MeOH. After 6h, solution was clear red. NaBH₃CN (28mg, 0.444mmol) was then added. The day after, solution had become brown. Reaction was stopped by addition of 0.5mL of H₂O and 0.5mL of HCI 1M. Solvent was removed and crude was purified by reverse phase chromatography with a gradient of ACN (0.1% TFA) and water (0.1% TFA). 8.5mg of brown solid was obtained as **3GG** pure product (Purity => 97.4%, Yield =>16%).
**¹H RMN (H₂O/D₂O)**: δ 8.06 (2H, dd), 7.82 (2H, dd), 7.48 (6H, m), 7.3 (2H, d), 4.33 (4H, s), 3.05 (4H, tr), 2.93 (8H, m), 1.99 (4H, m), 1.57 (4H, m).
¹³**C RMN (H₂O/D₂O)**: δ 150.9, 135.0, 128.1, 127.8, 127.4, 126.3, 124.9, 121.4, 121.2, 113.1, 46.8, 44.4, 43.6, 22.6, 22.4.
**MS:** Calculated for C₃₂H₄₀N₄O₂: 514.3308; found: 515.3438 (M + H)⁺

### Compounds of 3R1R2 type

### 2-(16-(2-hydroxyphenyl)-2,6,11,15-tetraazahexadecyl)naphthalen-1-οl (3AG)

**3A** (53mg, 0.171mmol) was solved in 15mL of THFₐₙₕ at 0 ºC. 1-hydroxy-2-naphthaldehyde (35mg, 0.205) was added solved in 1mL of THFₐₙₕ. After 4h, NaBH₃CN (24mg, 0.377mmol) was added and the reaction stirred overnight. The day after, reaction was stopped by addition of 0.5mL of H₂O and 0.5mL of HCI 1M. Solvent was removed and crude purified by reverse phase chromatography with a gradient of ACN (0.1 % TFA) and water (0.1% TFA). 32mg of **3AG** were obtained as a light brown powder (Purity 97.4%, Yield => 40%)
**¹H RMN (H₂O/D₂O)**: δ 8.06 (1H, dd, J1= 3.4Hz, J2=6.3Hz); 7.82 (1H, dd, J1= 3.4Hz, J2=6.3Hz); 7.49 (3H, m), 7.31 (1H, d, J3=8.4Hz3), 7.22 (2H, m,), 6.85 (2H, m), 4.32 (2H, s), 4.11 (2H, s), 3.00 (12H, m), 1.99 (4H, m), 1.59 (4H, m)
¹³**C RMN (H₂O/D₂O)**: 155.0, 150.9, 135.0, 131.7, 131.5, 128.1, 127.8, 127.4, 126.3, 125.1, 121.4, 121.2, 120.5, 117.0, 115.5, 113.1, 46.9, 44.4, 43.6, 22.6, 22.4.
**MS:** Calculated for C₂₃H₄₉N₄O₂: 464.3151; found MS => 465.3228 (M + H)⁺

### 1-(16-(2-hydroxyphenyl)-2,6,11,15-tetraazahexadecyl)naphthalen-2-ol (3AF)

**3A** (20mg, 0.065mmol) was solved in 10mL of THFₐₙₕ at 0 ºC. 2-hydroxy-1-naphthaldehyde (13mg, 0.078mmol) was added solved in 1mL of THFₐₙₕ. After 4h, NaBH₃CN (9mg, 0.143mmol) was added and the reaction stirred overnight. The day after, reaction was stopped by addition of 0.5mL of H₂O and 0.5mL of HCl 1M. Solvent was removed and crude purified by reverse phase chromatography with a gradient of ACN (0.1% TFA) and water (0.1% TFA). 6mg of **3AF** were obtained as a light brown powder (Purity >99%, Yield => 19%)
**¹H RMN (H₂O/D₂O)**: δ 7.90 (3H, m), 7.57 (1H, dd, J=8Hz), 7.4 (1H, dd, J=8Hz), 7.29 (2H, m), 7.21 (1H, d, J=9Hz), 6.91 (2H, m), 4.68 (2H, s), 4.19 (2H, s), 3.17 (2H, m), 3.02 (10H, m), 2.08 (4H, m), 1.66 (4H, m).
¹³**C RMN (H₂O/D₂O)**: δ 155.1, 154.2, 132.5, 132.2, 131.7, 131.6, 129.0, 128.5, 127.9, 123.8, 121.3, 120.6, 117.8, 117.1, 115.6, 114.9, 108.3, 46.9, 44.5, 43.9, 43.7, 41.7, 22.7, 22.5, 22.4.
**MS:** Calculated for C₂₃H₄₉N₄O₂: 464.3151; found: 465.3169 (M + H)⁺

### 2-(16-(9H-fluoren-3-yl)-2,6,11,15-tetraazahexadecyl)phenol (3AC)

**3A** (30 mg, 0.097 mmol) was solved in 5mL of Methanol at 0 ºC. 9H-fluorene-2-carbaldehyde (110 mg, 0.580 mmol) was added solved in 15mL of Methanol. The day after, NaBH₃CN (70 mg, 1.15 mmol) was added and the reaction stirred overnight. Reaction was stopped by addition of 2 mL of H₂O. Solvent was removed and crude purified by reverse phase chromatography with a gradient of ACN (0.1 % TFA) and water (0.1% TFA). 5mg of **3AC** were obtained as a white powder (Purity >99%, Yield => 12%)
**¹H RMN (H₂O/D₂O)**: δ 7.82 (2H, m), 7.56 (2H, m), 7.25 (3H, m), 7.21 (2H, m), 6.88 (2H, m), 4.18 (2H, s), 4.14 (2H, s), 3.85 (2H, s), 3.00 (12H, m), 2.01 (4H, m), 1.62 (4H, m).
¹³**C NMR (H₂O/D₂O)**: δ 155.0, 144.4, 143.9, 142.5, 140.2, 131.7, 131.6, 128.7, 128.6, 127.7, 127.1, 126.6, 120.5, 120.4, 120.3, 117.7, 115.5, 114.5, 51.4, 46.9, , 44.4, 43.6, 36.3, 22.6, 22.4.
**MS:** Calculated for C₃₁H₄₂N₄O: 486.7040; found: 487.4005 (M + H)⁺

### 2-(16-(naphthalen-2-yl)-2,6,11,15-tetraazahexadecyl) (3AN)

**3A** (15 mg, 0.0485 mmol) was solved in 5mL of THFₐₙₕ at 0ºC. 2-naphtaldehyde (9 mg, 0.0582 mmol) was added solved in 1mL of THFₐₙₕ. After 4h, NaBH₃CN was added and the reaction stirred overnight. The day after, reaction was stopped by addition of 0.5mL of H₂O and 0.5mL of HCl 1M. Solvent was removed and crude purified by reverse phase chromatography with a gradient of ACN (0.1% TFA) and water (0.1% TFA). 15mg of **3AN** were obtained as a white powder (Purity >99%, Yield => 69%)
**¹H RMN (H₂O/D₂O)**: δ 7.87 (m, 4H), 7.50 (m, 2H7.43 (d, J=8.44Hz, 1H), 7.22 (m, 2H), 6.85 (tr, J=6.97Hz, 2H), 4.3 (s, 2H), 4.12 (s, 2H), 3.00 (m, 12H), 1.98 (m, 4H), 1.59 (m, 4H).
¹³**C RMN (H₂O/D₂O)**: δ 155.0, 133.1, 132.7, 131.7, 131.5, 129.6, 129.1, 128.0, 127.7, 127.4, 127.0, 126.4, 120.5, 117.0, 115.5, 51.3, 46.9, 46.8, 44.4, 44.3, 43.7, 43.6, 22.6, 22.5, 22.4.
**HPLC:** tᵣ => 8.6min, Purity at 220nm => >99%
**MS:** Calculated for C₂₈H₄₀N₄O: 448.3202; found: 449.3520 (M + H)⁺

### 4-bromo-2-methoxy-6-(16-(naphthalen-1-yl)-2,6,11,15-tetraazahexadecyl)phenol (3LO)

**3O** (15mg, 0.036mmol) was solved in 5mL of THFₐₙₕ at 0ºC. 1-naphthaldehyde (6µL, 0.043mmol) was added solved in 1mL of THFₐₙₕ. The day after, NaBH₃CN (4.5mg, 0.072mmol) was added and the reaction stirred overnight. Reaction was stopped by addition of 0.5mL of H₂O and 0.5mL of HCl 1M. Solvent was removed and crude purified by reverse phase chromatography with a gradient of ACN (0.1% TFA) and water (0.1% TFA). 7mg of **3LO** were obtained as a brownish powder (Purity 98.7%, Yield => 35%)
**¹H RMN (H₂O/D₂O)**: δ 7.90 (3H, m, H7, H8, H9), 7.50 (4H, m, H2, H3, H4, H5), 7.14 (1H, d J=4Hz, H24), 6.97 (1H, d, J=4Hz, H26), 4.65 (2H, s, H11), 4.08 (2H, s, H22), 3.73 (3H, s, H29), 3.12 (2H, m, H15), 2.96 (10H, m, H12, H14, H18, H19, H21), 1.98 (4H, m, H13, H20), 1.59 (4H, m, H16, H17)
¹³**C RMN (H₂O/D₂O)**: δ 148.4 (C28), 144.1 (C27), 133.6 (C10), 130.8 (C6), 130.6 (C9), 129.5 (C5), 129.1 (C8), 127.5 (C3), 126.7(C4), 126.3 (C1), 125.6 (C2), 125.3 (C24), 122.4 (C7), 118.6 (C23), 116.7 (C26), 111.2 (C25), 56.4 (C29), 48.1 (C11), 46.9 (C14), 45.9 (C22), 44.3 (C15, C18, C19, C21), 22.7 (C16, C17), 22.6 (C13), 22.4 (C20).
**MS:** Calculated C₂₉H₄₁BrN₄O₂: 557.5770; found 557.2440 and 559.2421 (M + H)⁺

### 1-(16-(5-bromo-2-hydroxy-3-methoxyphenyl)-2,6,11,15-tetraazahexadecyl)naphthalen-2-ol (3FO)

**3O** (15mg, 0.036mmol) was solved in 5mL of THFₐₙₕ at 0 ºC. 2-hydroxy-1-naphthaldehyde (7mg, 0.043mmol) was added solved in 1mL of THFₐₙₕ. The day after, NaBH₃CN (4.5mg, 0.072mmol) was added and the reaction stirred overnight. Reaction was stopped by addition of 0.5mL of H₂O and 0.5mL of HCl 1M. Solvent was removed and crude purified by reverse phase chromatography with a gradient of ACN (0.1 % TFA) and water 0.1% TFA). 5mg of **3FO** were obtained as a white powder (Purity >99%, Yield => 25%)
**¹H RMN (H₂O/D₂O)**: δ 7.86 (3H, m, H3, H5, H8), 7.54 (1H, dd, H6, J=7.5Hz), 7.36 (1H, dd, H7, J=7.5Hz), 7.18 (2H, m, H2, H24), 7.01 (1H, d, H26, J=2Hz), 4.64 (2H, s, H11), 4.12 (2H, s, H22), 3.88 (3H, s, H29), 3.12 (2H, m, H12, H21), 3.00 (10H, m, H14, H15, H18, H19) 2.03 (4H, m, H13, H20), 1.63 (4H, m, H16, H17)
¹³**C RMN (H₂O/D₂O)**: δ 154.2, 148.4, 144.4, 132.5, 132.1, 129.0, 128.4, 127.9, 125.2, 123.7, 121.2, 117.3, 116.6, 114.8, 111.2, 108.2, 56.3, 46.9, 44.4, 43.8, 41.6, 22.6, 22.5.
**MS:** Calculated C₂₉H₄₁BrN₄O₃: 573.5760; found: 575.2441 and 573.2471 (M + H)⁺

### 1-(16-(4-hydroxyphenyl)-2,6,11,15-tetraazahexadecyl)naphthalen-2-ol (3FJ)

**3J** (23mg, 0.074mmol) was solved in 10mL of THFₐₙₕ at 0 ºC. After that, 2-hydroxy-1-naphthaldehyde (15mg, 0.089mmol) was added solved in 5mL of THFₐₙₕ. The day after, NaBH₃CN (9mg, 0.148mmol) was added and the reaction was stirred overnight. Reaction was stopped by the addition of 1mL of H₂O and 1mL of HCI 1M. Solvent was removed and crude was purified by reverse phase chromatography with a gradient of ACN (0.1% TFA) and water (0.1% TFA). 28 mg of **3FJ** were obtained as a white fluffy solid (Purity => 98.5%, Yield 82%).
**¹H RMN (H₂O/D₂O)**: δ 7.81 (3H, m), 7.5 (1H, dd, J1=7.5Hz, J2=1Hz), 7.32 (1H, dd, J1=7.5Hz, J2=1Hz), 7.19 (2H, d, J3=8.5Hz), 7.14 (1H, d, J5=8Hz), 6.79 (2H, d, J3=8.5Hz), 4.6 (inside water, s), 4.02 (3H, s), 3.09 (2H, m), 2.95 (10H, m), 1.95 (4H, m), 1.58 (4H, m).
¹³**C RMN (H₂O/D₂O)**: δ 156.7, 154.2, 132.4, 132.1, 132.0, 131.6, 129.0, 128.4, 127.8, 123.7, 121.2, 117.3, 115.9, 108.2, 50.6, 46.9, 44.4, 43.7, 43.4, 41.7, 40.0, 22.6, 22.5.
**MS:** Calculated C₂₃H₄₉N₄O₂: 464.3151; Found MS => 465.3310 (M + H)⁺

### Biophysical and biochemical tests related to the inhibition of the heparin activity.

**Binding of the spermine derivatives to the heparin target.** Surface Plasmon Resonance (SPR) on heparin-functionalized chips were used to determine the apparent dissociation constant of selected molecules (*K*_{D}^{app}, Table 1). The SPR-determined *K*_{D}^{app} for molecules containing an *ortho* phenol and a large aromatic ring (3AF and 3AG) display a *K*_{D}^{app} in the low micromolar range (entries 1 and 2). Quite remarkably, 3FF and 3AC render sub-micromolar affinity to Hep by SPR (entries 3 and 4). Moreover, two of the identified hits (3AC and 3FF) show higher affinity to Hep than ciraparantag (entry 5).

**Table 1. Apparent dissociation constants, K_{D}^{app} (µM), for the interaction of selected molecules with Hep.**

| | | K_{D}^{app} (µM) |
|---|---|---|
| Entry | Binder | SPR |
| 1 | 3AF | 2.62±0.20 |
| 2 | 3AG | 1.86±0.12 |
| 3 | 3FF | 0.797±0.24 |
| 4 | 3AC | 0.567±0.013 |
| 5 | Ciraparantag | 1.53±0.12 |

Many binders are fluorescent and their emission spectra are perturbed by the interaction with Hep, allowing to obtain additional information about the supramolecular complexes in solution. The emission spectrum of 3AC is strongly reduced upon addition of up to 2 equivalents of the Hep disaccharide repeating units, slightly recovering upon additional Hep (Figure 1A). Despite the complex titration isotherm, this could be fitted to a reasonable binding mode considering the disaccharide repeating unit of Hep as the binding motif (inset in Figure 1A). The proposed binding model is depicted in Figure 1B: 3AC forms a strong complex with one ligand per disaccharide repeating unit ([3AC-Hep] complex), which evolves to the [3AC-Hep₂] complex upon additional Hep, or to a less specific species [3AC₂-Hep] in the presence of excess 3AC. This binding model explains the observed fluorescence emission results: the proximity of the chromophores in the [3AC-Hep] and [3AC₂-Hep] complexes accounts for the fluorescence quenching that is partially recovered when the 3AC bound molecules are spread along the Hep chain in the [3AC-Hep₂] species. A putative *K*_{D}^{app} ≈ 0.5 µM can be estimated from the fluorescence binding data, which is in reasonable agreement with the SPR results.

The 3AC-Hep binding was also studied using ¹H NMR, in this case with a shorter heparin oligomer, dp14, which on average contains seven disaccharide repeating units. The titration of 3AC with dp14 (Figure 1C) shows two different phases. At the beginning of the titration (low dp14 : 3AC ratio), it has been observed a large broadening of the 3AC spectrum leading to the disappearance of some proton signals. Under these conditions, the [3AC₂-dp14] and [3AC-dp14] complexes prevail (see Figure 1B), which lead to an electrostatic charge compensation of dp14 favoring the aggregation of the species in solution. Addition of more equivalents of Hep produces the complete formation of [3AC-dp14₂] species, which is clearly visible in the ¹H NMR spectrum by the growing of broad signals at a different chemical shift from those of 3AC alone. This titration experiment strongly correlates with the binding mode inferred from the fluorescence titration, while the slow exchange in the chemical shift NMR timescale is consistent with the very strong binding quantified by SPR and fluorescence spectroscopy.

**In vitro inhibition of heparin activity.** The effect of the binders as Hep antidotes can be tested in vitro using an enzymatic reaction related to the blood coagulation process (Figure 2A). This test employs human recombinant coagulation factor Xa (FXa) and antithrombin III (AT_{III}), with the synthetic peptide Z-D-LGR-N(Me)ANBA as the substrate of the FXa protease to mimic the prothrombin to thrombin transformation that triggers blood coagulation. Heparin forms a ternary complex with AT_{III} and FXa with a low hydrolytic activity, as a model for the anticoagulation effect of Hep. The addition of an efficient Hep binder dissociates the [FXa-Hep-AT_{III}] complex, thus releasing the active form of FXa that cleaves the substrate. The peptide hydrolysis can be readily monitored by analytical HPLC thanks to the ANBA chromophore, as an accurate measurement of the reaction kinetics.

Figure 2B shows the kinetic of hydrolysis of the peptide in different reaction conditions. In the absence of Hep (solid circles) the FXa exhibits the highest activity, which is clearly inhibited when Hep is present (0.1 IU/mL, solid squares). In parallel experiments, the addition of equal concentration (1 µM) of each of the binders produces a different level of recovery of the FXa activity. Thus, the in vitro potency as Hep antidote grows in the series 3AL (X symbols) ≤ 3AG (rhombuses) < 3AF (open circles) < 3FF (inverted solid triangles) < 3AC (solid triangles). This trend nicely correlates with the binding data shown in Table 1. Actually, several selected molecules derived from spermine were tested in the enzymatic assay (Figure 2C) most of them showing activity in the reversal of the heparin action. Also in the in vitro assays, 3FF and 3AC perform as the most active Hep antidotes, with 3FF being comparable with the drug ciraparantag (cirap.) and 3AC showing to be even more potent (Figure 2C).

**Reversing the anticoagulant activity of heparin:** In order to check the efficacy of the antidotes in a more representative environment, 3FF and 3AC were tested in real blood coagulation assays. Thus, it has been monitored the coagulation of freshly extracted mouse blood in the absence or presence of Hep, and upon addition of different concentrations of 3FF and 3AC. By simply turning around the vials after 15 minutes (Figure 3A), the differences in their viscosity are evident: the control samples are completely coagulated, Hep-containing vials remain liquid (the blood flows down), while 3FF and 3AC are able to revert the anticoagulant effect of Hep in a dose-response manner. Actually, at least qualitatively, 3AC seems to be a better antidote than 3FF in this simple ex vivo experiment, since a more robust clot was observed at equal doses.

3FF and 3AC were tested in an in vivo coagulation model. It was thus performed the tail transection assay with mice after consecutive injection of Hep, and the drugs at two different doses each (Figure 3B). The control groups injecting saline or Hep, and those corresponding to just the drugs were also included. The results clearly show that both 3FF and 3AC are efficient antidotes of Hep also in vivo, strongly reducing the bleeding of heparinized mice. Remarkably, the amount of blood loss is dose responsive for both drugs, and 3AC is a more potent reversal agent than 3FF. At the highest doses of 3FF/3AC here used, the blood loss of heparinized mice was reversed to values very similar to those of the control group injected with saline, strongly supporting the potential use of these molecules as Hep antidotes.

## Claims

1. A compound of Formula I: 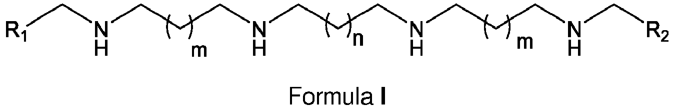 wherein:
n is an integer between 0 and 3;
each m is independently an integer between 0 and 3;
R₁ is phenyl, naphthyl or fluorenyl, wherein R₁ is optionally substituted by one to three groups R₃;
R₂ is naphthyl or fluorenyl, wherein R₂ is optionally substituted by one to three groups R₃;
each R₃ is independently -OH, -OR₄, halogen, -C₁₋₄ alkyl, -CHO or -OPO(OH)₂; and
R₄ is C₁₋₄ alkyl,
with the proviso that:
if R1 is 2-methoxyphenyl, 2-hydroxyphenyl or 2-methoxy-1-naphthalen-1-yl, n=2 and m=1, then R2 is not 1-naphthyl or 2-methoxy-1-naphthalen-1-yl; and
the compound of Formula **I** is not
N1,N1'-(butane-1,4-diyl)bis(N3-(naphthalen-1-ylmethyl)propane-1,3-diamine, or
N1,N1'-(butane-1,4-diyl)bis(N3-(naphthalen-2-ylmethyl)propane-1,3-diamine.

2. The compound of Formula **I** according to claim 1, wherein n is 2.

3. The compound of Formula **I** according to any of claims 1 or 2, wherein m is 1.

4. The compound of Formula **I** according to any of claims 1 to 3, wherein R₄ is -CH₃.

5. The compound of Formula **I** according to claim 1, selected from:

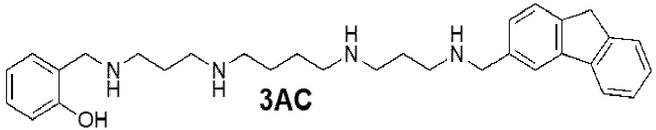

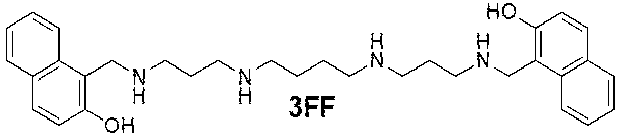

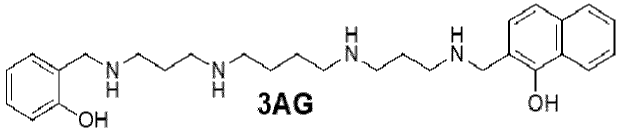

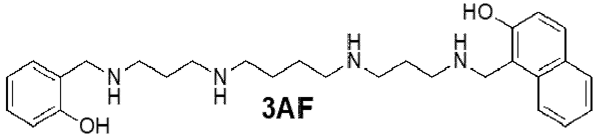

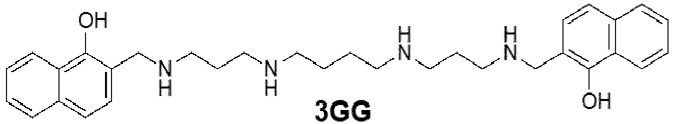

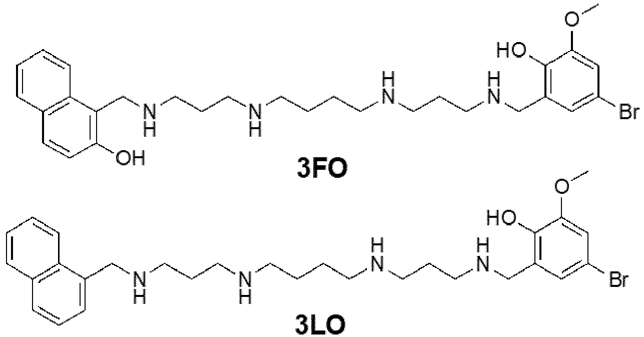

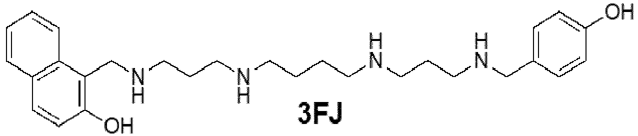

6. The compound of Formula **I** according to claim 5 selected from **3AC, 3FF, 3AG** and **3AF.**

7. A pharmaceutical composition which comprises a compound of Formula **I** according to any of claims 1 to 6 or a pharmaceutically acceptable salt thereof, and one or more pharmaceutically acceptable excipients.

8. A compound of Formula **I** according to any of claims 1 to 6 or a pharmaceutically acceptable salt thereof, for use as a medicament.

9. A compound of Formula **I** according to any of claims 1 to 6 or a pharmaceutically acceptable salt thereof, for use as antidote of heparin in the control of blood coagulation.
